Europäisches Patentamt

European Patent Office

Office européen des brevets

(11) Publication number: **0 220 948**
**A1**

## (12) EUROPEAN PATENT APPLICATION

(21) Application number: 86308264.0

(22) Date of filing: 23.10.86

(51) Int. Cl.⁴: **C 07 C 50/04**
C 07 C 46/06, C 07 C 37/07,
C 07 C 39/08

(30) Priority: 29.10.85 GB 8526630

(43) Date of publication of application:
06.05.87 Bulletin 87/19

(84) Designated Contracting States:
BE CH DE FR GB IT LI NL

(71) Applicant: The British Petroleum Company p.l.c.
Britannic House Moor Lane
London EC2Y 9BU (GB)

(72) Inventor: Griggs, Colin George
The British Petroleum Company p.l.c. Chertsey Road
Sunbury-on-Thames Middlesex, TW16 7LN (GB)

(74) Representative: Fawcett, Richard Fennelly et al
BP INTERNATIONAL LIMITED Patents Division Chertsey
Road
Sunbury-on-Thames Middlesex, TW16 7LN (GB)

(54) **Process for the preparation of quinones.**

(57) A process for the production of quinones from an aromatic alcohol is described. The process uses a hydrocarbyl hydroperoxide, e.g t-butylhydroperoxide, and a copper catalyst to effect the reaction. The use of a hydrocarbyl hydroperoxide improves the rate of reaction relative to the use of hydrogen peroxide. The process is particularly useful as a first stage in the production of 2, 3, 6-trimethylhydroquinone, a Vitamin E precursor, from cheap, readily available starting materials.

EP 0 220 948 A1

**Description**

PROCESS FOR THE PREPARATION OF QUINONES

The present invention relates to a process for the preparation of quinones, and in particular to the preparation of quinones which are themselves suitable precursors for the manufacture of compounds possessing Vitamin E activity.

Compounds which possess Vitamin E activity are generally termed tocopherols. Although such compounds occur naturally in, for example corn oils, wheat oil and safflower oil, world demand for such materials means that it is desirable to manufacture these compounds on a large scale commercially.

Synthetic forms of the naturally occuring tocopherols have been developed and the production ofα-tocopherol, for example, is operated commercially. One method of preparingα-tocopherol commercially is to condense the alkene isophytol with trimethyl-1. 4-benzoquinone in the presence of an acidic catalyst such as zinc chloride or boron trifluoride in the liquid phase. The tocopherol is obtained on aqueous work up.

A major problem in the commercial exploitation of this process is the relatively high cost of trimethyl-1,4-benzoquinone or the related 2,3,5-trimethylhydroquinone which is reflected in the final price of the tocopherol. The high cost arises because there are few simple and selective methods of preparing these compounds on a large scale from cheap starting materials.

A possible method of preparing trimethyl-1,4-benzoquinone is described in Tetrahedron Letters 24 5249-5252 (1983). According to this paper, oxidation of the phenol 2,3,6-trimethylphenol with hydrogen peroxide occurs in the presence of a ruthenium catalyst to produce the corresponding quinone in high yields and, more importantly, with high selectivity. The product quinone can then be reduced, using well known methods, to 2,3,6-trimethylhydroquinone. The process is limited to the use of a ruthenium catalyst since related metals, e.g. Fe, Cu and Mo, all lead to unselective oxidation.

The process as described in this reference has the disadvantage that the reaction is very slow. Thus although 90% yields of the quinone can be obtained during the reaction the residence times approach 5 hours.

A process has now been identified which allows aromatic alcohols, and in particular 2,3,6-trimethylphenol or 2,3,5-trimethylphenol, to be oxidised selectively to the corresponding quinone at rates faster than those obtained in the prior art. The process comprises the use of a copper catalyst in the presence of a hydrocarbyl hydroperoxide to effect the oxidation process.

Accordingly, the present invention provides a process for the production of quinones, which process comprises contacting an aromatic alcohol with an oxidant in the presence of a copper catalyst under oxidation conditions characterised in that the oxidant is a hydrocarbyl hydroperoxide.

An aromatic alcohol is defined as an alcohol having a hydroxyl group directly joined to an aromatic ring. Suitable aromatic alcohols which can be used are phenols, for example phenol itself, the isomeric cresols, xylenols, napthols and the like. A particularly preferred feedstock is an alkyl substituted phenol most preferably 2,3,6-trimethylphenol or 2,3,5-trimethylphenol. Accordingly, an embodiment of the invention described comprises a process for the production of the quinone of 2,3,6-trimethylphenol or 2,3,5-trimethylphenol which process comprises contacting 2,3,6-trimethylphenol or 2,3,5-trimethylphenol and an oxidant with a weak acid and a copper catalyst under oxidation conditions characterised in that the oxidant is a hydrocarbyl hydroperoxide.

As regards the hydrocarbyl hydroperoxide, this is suitably an alkyl, cycloalkyl, aralkyl, alkaryl or aryl hydroperoxide. Hydrocarbyl hydroperoxides having substituted alkyl or aryl groups can also be used. Preferred hydrocarbyl hydroperoxides are t-butylhydroperoxide, cumene hydroperoxide, cyclohexylhydroperoxide and the like. The hydrocarbyl hydroperoxide can be used undiluted, or as a solution in water or a solvent which does not undergo substantial oxidation under the reaction conditions. Preferred solvents include tertiary alcohols, aromatics compounds, e.g. toluene, benzene, xylene, chlorobenzene, and $C_5$-$C_{12}$ alkanes.

Although a wide range of hydrocarbyl hydroperoxide concentrations can be used it is preferable that the hydrocarbyl hydroperoxide is present in molar excess over the aromatic alcohol.

By the term quinone is meant an aromatic compound in which two hydrogen atoms connected to the aromatic ring are replaced by two oxygen atoms. The two oxygen atoms are disposed meta- ortho- or para- to each other on the aromatic ring.

By the term hydroquinone is meant a quinone in which the two oxygen atoms have been converted into hydroxyl groups.

a A copper catalyst is used in order to accelerate the reaction rate and to obtain the quinone free from substantial amounts of side products. The catalyst may be a copper(I) salt, a copper(II) salt or a mixture thereof. The salts are for preference ones which are soluble in the reaction mixture. Suitable copper(I) and copper(II) salt catalysts include the simple inorganic salts, e.g. CuCl and $CuCl_2$, as well as coordination complexes of copper with ligands such as amines, phosphines, stibines, arsines, porphyrins, corrins, phthalocyanins etc. Preferably, the copper catalyst is a copper(I) or copper(II) halide e.g. a copper chloride or bromide. The copper catalyst is preferably added in amounts up to 10% by weight of the aromatic alcohol used. When copper catalysts other than copper(I) or copper (II) halides are used, it is preferable to add a source of halide, e.g. a hydrohalic acid with the particular copper catalyst used. The copper catalyst may be generated in situ during the process from, for example, finely divided copper metal.

The process is preferably carried out in a weak acid. A weak acid is defined as an acid having a pKa, as measured at 25°C in water, of greater than 0. Suitably the weak acid is an organic acid for example a $C_2$ to $C_6$ aliphatic carboxylic or dicarboxylic acid. Preferred acids include acetic acid. propanoic acid. formic acid. succinic acid, malic acid, butanoic acid, trifluoroacetic acid and the like. The role of the weak acid is. in combination with the hydrocarbyl hydroperoxide, to maintain high rates of reaction. It is preferable to add the weak acid component in amounts such that it is in a molar excess over at least one of the reactants.

The oxidation of the aromatic alcohol to the corresponding quinone occurs readily at room temperature although higher temperatures can be used to accelerate the reaction rate further. However in operating at high temperatures, it must be remembered that the rate of thermal decomposition of the hydrocarbyl hydroperoxide also increases leading to a less efficient use of the oxidant. Furthermore the rate of side product formation becomes more important and the selectivity to the quinone decreases. With these constraints in mind it is preferable to operate the process in the temperature range 10 to 200°C, preferably 20 to 100°C.

The process as described may be operated batchwise, but by virtue of the short reaction times may also be operated continuously.

The quinone produced by the process may be converted into its hydroquinone by, for example, reduction with sodium hydrosulphite, sulphur dioxide in water, or a catalyst such as Raney Nickel and hydrogen. Such processes will be familiar to the skilled man. It is therefore possible to convert an aromatic alcohol into a hydroquinone using a two step process.

Accordingly, in an aspect of this invention there is provided a process for the preparation of a hydroquinone from an aromatic alcohol characterised in that

(a) in a first stage the aromatic alcohol is converted into its corresponding quinone by oxidation with a hydrocarbyl hydroperoxide in the presence of an organic acid and a copper catalyst, and

(b) in a second stage the quinone is reduced to the hydroquinone using a reducing agent.

The quinone may be optionally isolated from the reaction mixture after the first stage e.g. by distillation or sublimation or it may be reduced in situ to the hydroquinone, which itself can be subsequently isolated and used subsequently. The second stage can be effected, for example, by reducing with a reducing agent such as sodium bisulphite; Raney nickel and hydrogen; or palladium/carbon and hydrogen.

The invention as described is now illustrated by the following Examples.

Examples 1-5

A mixture of the appropriate copper compound (50mg), glacial acetic acid (100ml) and 2,3,6-trimethylphenol (5g, 37 mmol) were stirred in a water bath at 23°C. To this solution was added dropwise, over 5 min t-butylhydroperoxide (TBHP) in t-butanol (TBA) (17.5ml of a 4.5M solution, 79mmol, 2.1 equivalents). In the case of copper(I) salts the addition was done under nitrogen. The reaction, after complete addition of the oxidant, was periodically monitored by tlc ($CH_2Cl_2Al_2O_3$ plates) for disappearance of the starting phenol. The product in each experiment was analysed after complete consumption of the phenol and the result is given in Table 1.

| Example | Catalyst | Quinone Yield (A) /% | Reaction (B) Time |
|---------|----------|----------------------|-------------------|
| 1 | CuCl | 95 | 20 min |
| 2 | $CuCl_2$ | 91 | 15 min |
| 3 | $Cu(NO_3)_2$ | 54 | 30 h |
| 4 | $Cu(acac)_2$ | 45 | 22 h |
| 5 | $CuSO_4$ | 39 | 22 h |

### TABLE 1

A = Yields of quinone (trimethyl-1,4-benzoquinone) are for products purified, after aqueous work-up, by column chromatogrophy ($SiO_2$).

B = Reaction time is for complete consumption of the starting phenol after complete addition of the oxidant.

Examples 6-8

The appropriate weight of copper(II) chloride, glacial acetic acid (100ml) and 2,3,6-trimethylphenol (5g, 37 mmol) were stirred in a water bath at 23°C. To this solution was added, over 5 min dropwise, t-butyhydroperoxide (TBHP) in t-butanol (TBA) (17.5ml of a 4.5M solution, 79mmol, 2.1 equivalents). The reaction, after complete addition of the oxidant, was periodically monitored by tlc ($CH_2Cl_2Al_2O_3$ plates) for disappearance of the starting phenol. The product in each experiment was analysed after complete consumption of the phenol and the result obtained for the respective weight of $CuCl_2$ used is given in Table 2.

| Examples | Catalyst | Catalyst wt/mg | Quinone Yield (A) /% | Reaction (B) Time |
|----------|----------|----------------|----------------------|-------------------|
| 6 | $CuCl_2$ | 50 | 91 | 15 min |
| 7 | $CuCl_2$ | 25 | 64 | 15 min |
| 8 | $CuCl_2$ | 10 | 34 | 15 min |

### TABLE 2

A,B are as defined for Table 1.

## Claims

1. A process for the production of quinones which process comprises contacting an aromatic alcohol with an oxidant in the presence of a copper catalyst characterised in that the oxidant is a hydrocarbyl hydroperoxide.

2. A process as claimed in Claim 1 characterised in that the hydrocarbyl hydroperoxide is an alkyl, cycloalkyl, aralkyl or aryl hydroperoxide.

3. A process as claimed in Claim 2 characterised in that the hydrocarbyl hydroperoxide is cumene hydroperoxide or cyclohexyl hydroperoxide.

4. A process as claimed in Claim 2 characterised in that the hydrocarbyl hydroperoxide is t-butylhydroperoxide.

5. A process as claimed in claim 1 characterised in that it is carried out in the presence of a weak acid having a pKa of greater than 0 at 25°C in water.

6. A process as claimed in claim 5 characterised in that the weak acid is acetic acid.

7. A process as claimed in claim 5 characterised in that the weak acid is selected from propanoic acid, formic acid, butanoic acid, succinic acid, malic acid and trifluoroacetic acid.

8. A process as claimed in claim 1 characterised in that the aromatic alcohol is a phenol, cresol, xylenol or napthol.

9. A process as claimed in Claim 8 characterised in that the aromatic alcohol is an alkyl substituted phenol.

10. A process as claimed in Claim 9 characterised in that the aromatic alcohol is 2,3,6-trimethylphenol or 2,3,5-trimethylphenol.

11. A process for the preparation of a hydroquinone from an

   (a) in a first stage the aromatic alcohol is converted into its corresponding quinone by oxidation with a hydrocarbyl hydroperoxide in the presence of a weak acid and a copper catalyst, and
   (b) in a second stage the quinone is reduced to the hydroquinone using a reducing agent.

## DOCUMENTS CONSIDERED TO BE RELEVANT

EP 86308264.0

| Category | Citation of document with indication, where appropriate, of relevant passages | Relevant to claim | CLASSIFICATION OF THE APPLICATION (Int. Cl.4) |
|---|---|---|---|
| X | US - A - 4 491 545 (FRANK THOEMEL et al.)<br><br>* Claim 1; examples 2,3,5; column 1, lines 29-31 *<br><br>-- | 1,2,4, 5,8-11 | C 07 C 50/04<br>C 07 C 46/06<br>C 07 C 37/07<br>C 07 C 39/08 |
| D,A | TETRAHEDRON LETTERS, vol. 24, no. 47, 1983, Pergamon Press: Oxford, New York, Paris, Frankfurt<br><br>SATORU ITO et al. "Ruthenium-Catalyzed Oxidation of Phenols with Hydrogen Peroxide" pages 5249-5252<br><br>-- | 1,8-10 | |
| A | US - A - 4 522 757 (CHAO-YANG HSU et al.)<br><br>* Claims 1,13-15 *<br><br>-- | 1,8-10 | |
| P,A | EP - A1 - 0 175 574 (THE BRITISH PETROLEUM)<br><br>* Claims *<br><br>---- | 1-11 | **TECHNICAL FIELDS SEARCHED (Int. Cl.4)**<br><br>C 07 C 50/00<br>C 07 C 46/00<br>C 07 C 37/00<br>C 07 C 39/00 |

The present search report has been drawn up for all claims

| Place of search | Date of completion of the search | Examiner |
|---|---|---|
| VIENNA | 23-12-1986 | REIF |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03.82